# EUROPEAN PATENT SPECIFICATION

(11) **EP 1 061 986 B1**
(45) Date of publication and mention of the grant of the patent: **15.09.2004**
(21) Application number: 99932477.5
(22) Date of filing: 16.02.1999
(51) Int. Cl.: A61M 29/02

(54) **ANGIOPLASTY AND STENT DELIVERY CATHETER**
KATHETER ZUR DURCHFÜHRUNG EINER ANGIOPLASTIE UND ZUM EINBRINGEN EINES STENTS
CATHETER POUR ANGIOPLASTIE ET L'INTRODUCTION DE STENT

(30) Priority: 16.02.1998 EP 98200476; 04.05.1998 EP 98201420; 29.05.1998 US 86382
(43) Date of publication of application: 27.12.2000
(73) Proprietor: MEDICORP S.A., 54600 Villers-lès-Nancy (FR)
(72) Inventor: AMOR, Max, F-54000 Nancy (FR); FRID, Noureddine, B-1850 Beersel (BE); HENRY, Michel, F-54000 Nancy (FR); RUFENACHT, Daniel, CH-1206 Genève (CH)
(74) Representative: Vandeberg, Marie-Paule L.G.
(86) International application number: PCT/BE1999/000020
(87) International publication number: WO 1999/040964

(56) References cited:
- EP-A- 0 330 376
- WO-A-95/05209
- WO-A-96/39955
- US-A- 5 423 742

## Description

### Field of the invention

The present invention relates to a device for protected angioplasty, intended for the implantation of luminal endoprostheses (or stents) in critical areas such as carotid or vertebral arteries, where temporary protection of downstream-situated organs is highly desirable.

### Background of the invention

Angioplasty is now recognised as a highly valuable method for curing stenosis and other luminal diseases of the vascular system.

However, this technique, although quite appreciated and constantly improved, is not yet systematically used for treating each type of such diseases. In particular, the brain is a critical area, where vessels are very thin and where even short occlusions could lead to irreversible damages for the patient.

Vessels near the brain, as for instance the carotid bifurcation, are to be treated with high care because incident epiphenomena that could be considered as minor in other places could have there disastrous consequences for the patient. Because time is of the essence, the circulation of blood may not be interrupted without heavy consequences, due to the lack of oxygen in the brain. It is generally admitted that circulation may not be interrupted during more than a couple of minutes.

The carotid and the carotid bifurcation are furthermore, on a mechanical point of view, critical parts of the body.

The carotid bifurcation has a specific shape (including a segment of widened then restricted cross-section), which is known to provoke turbulence in the blood flow, leading to high local solicitation of the artery walls. As a consequence, stenosis problems are rather frequent in carotids.

A specific problem which occurs in treatment of stenosis in the inner carotid by angioplasty is the evacuation of debris that pile up in the stenosis. After the stenosis is cured, theses debris are naturally carried by the flowing blood. There is a strong risk that they be transported up downstream to the lesions, into capillary arteries that they could block, causing thrombosis with catastrophic embolization.

The carotid is considered by some physicians as the last frontier for endovascular therapy. There is still at present considerable scepticism regarding angioplasty in the carotid.

### Description of the prior art

It has thus proved desirable to improve this technique. Among recent improvements, methods for cerebral protection have been conceived and some of them are being developed. The "Report on 2nd Carotid Angioplasty Meeting, October 23 and 24, 1997, Polyclinique Essey-les-Nancy, France" summarises various aspects of the present advancement of research and development in the field of carotid angioplasty.

The technique developed by Théron to cure carotid stenosis with cerebral protection consists of introducing a triple coaxial catheter in the common carotid. A microcatheter provided with a latex balloon at its tips is inserted through the guiding catheter. The lesion is located and the micro-catheter is advanced through the stenosis, after which said balloon is inflated downstream of the lesion. An angioplasty balloon (or dilatation balloon) is inflated at the level of the stenosis. Particles originating from the stenosis are aspired and flushed through the guiding catheter with heparin, the flow being diverted towards the external carotid. The placement of a stent is then performed, the angioplasty balloon is withdrawn. A new aspiration and a flush are performed once again, while the protection balloon remains inflated. The latter is thereafter deflated and, if the result seems satisfactory, the micro-catheter, the protection balloon and the guiding catheter are removed.

This method, although attractive is regretfully not perfectly reliable.

Trials have shown that when using same, a non-neglectible amount of debris can remain in the blood flow.

Many of these debris do have too large a diameter to be efficiently destroyed by the white cells before reaching places in the brain where they could cause fatal thrombosis.

In fact, aspiration and flushing from upstream the protection balloon is not totally efficient, and furthermore necessitates that the tip of the catheter be approached as close as possible to the balloon, and that the operation be carried out under severe control. This is liable to cause problem inasmuch as time is of the essence in such kinds of interventions where many other aspects (treatment of the stenosis, placement of the stent) must also be taken into account in a very short time.

It has been suggested, for example in WO-95 05209 to use another technique designated as "double balloon technique", which would consist of occluding the carotid artery beyond the stenosis and also occluding the upper part of the common carotid artery, thus creating a dilatation chamber that could easily be aspirated and cleaned, or wherein various surgical or diagnosis instruments can be inserted afterwards. The various parts of the double-balloon device being each provided with a distinct operative fluid, this device is rather cumbersome and awkward to manoeuvre .

### Brief description of the invention

Another method and device for safely implanting a luminal endoprosthesis in critical areas like carotid, has been developed, according to which efficient protection of organs situated downstream of a carotid stenosis may be obtained without any necessity of aspiring the debris of the lesion.

The subject of the invention is a device for the implantation of expandable stents in a vascular vessel, allowing for temporary protection of downstream organs, Comprising - a central part provided with an axial lumen, for a microguide wire, comprising a microcatheter bearing at its distal end an atraumatic tip,-a tip balloon part comprising an inflatable occlusive balloon hermetically connectable via the axial lumen to injection means able to feed said balloon with a physiologically acceptable fluid wherein
- the central part comprises a stent pusher part) surrounded by a stent-releasing part,
- a stent loading cavity, containing a stent in radially contracted state, extends near the distal end of the stent pusher part,
- a fluid-releasing section extends at the proximal side of the occlusive balloon, said releasing section releasing in operation the same physiologically acceptable fluid fed at predetermined rates from the inflated occlusive balloon into an upstream section of the vessel when the pressure of said fluid resches a predetermined level.

According to an avantageous embodiment, this device comprises a dilatation balloon axially displaceable relative to the tip balloon.

The stent is preferably a self-expanding stent, which is maintained in place in the stent loading cavity by a surrounding shell.

The stent can also be a balloon-dilatable stent, the stent-releasing part comprising in that case a dilatation balloon.

The fluid-releasing section extends preferably at the proximal end of the occlusive balloon, advantageously on the neck of the balloon or on a cane (24) supporting the occlusive balloon.

The microguide wire may be anchored at the distal side of the occlusive balloon. In such an embodiment,the wire is preferably terminated by a ball inserted in a pouch provided at said distal side.

The fluid-releasing section is advantageously designed so that the fluid cannot escape unless its pressure reaches a trigger value.

The invention also relates to a method for the implantation of endoluminal stent in a vessel comprising operations as described in the present text.

Thanks to this method, the debris of the stenosis are flushed away in a very efficient manner and further deviated to other places of the body where they can provoke no harm, (for instance via the outer carotid in the case where carotid stenosis is cured).

### Brief description of the drawings

Other advantages of the device and of the method according to the invention will appear from the description hereafter of particular embodiments thereof, reference being made to the appended drawings wherein
Fig. 1 is a diagrammatic lateral view of a longitudinal cross-section of the distal end of a device according to the invention bearing a self-expanding endoprosthesis,
Fig.la is a cross section according to line II-II of the device of Fig.1,
Fig. 2 is a detailed view of the tip and of a distal end of the device of Fig. 1,
Fig. 3 is a diagrammatic lateral view in situ of a device according to Fig. 1,
Figs. 4 and 5 are diagrammatic lateral views of different embodiments of a device of the invention,
Figs 6 to 12 are diagrammatic views illustrating various steps of a method for using the device of the invention,
Figs. 13 and 14 illustrate optional steps of the above method,
Fig. 15 is a lateral view of another embodiment of the device of the invention,
Fig. 16 is a lateral view of still another embodiment of the invention.

### Detailed description of the drawings

In the present context, the term "stent" is used for the sake of facility to define generally speaking a luminal endoprosthesis, bearing in mind that luminal endoprostheses include stents sensu stricto but can comprise, together with their framework, various kinds of coating (not shown). It is clear that the invention also relates to devices and methods where such coated stents are used.

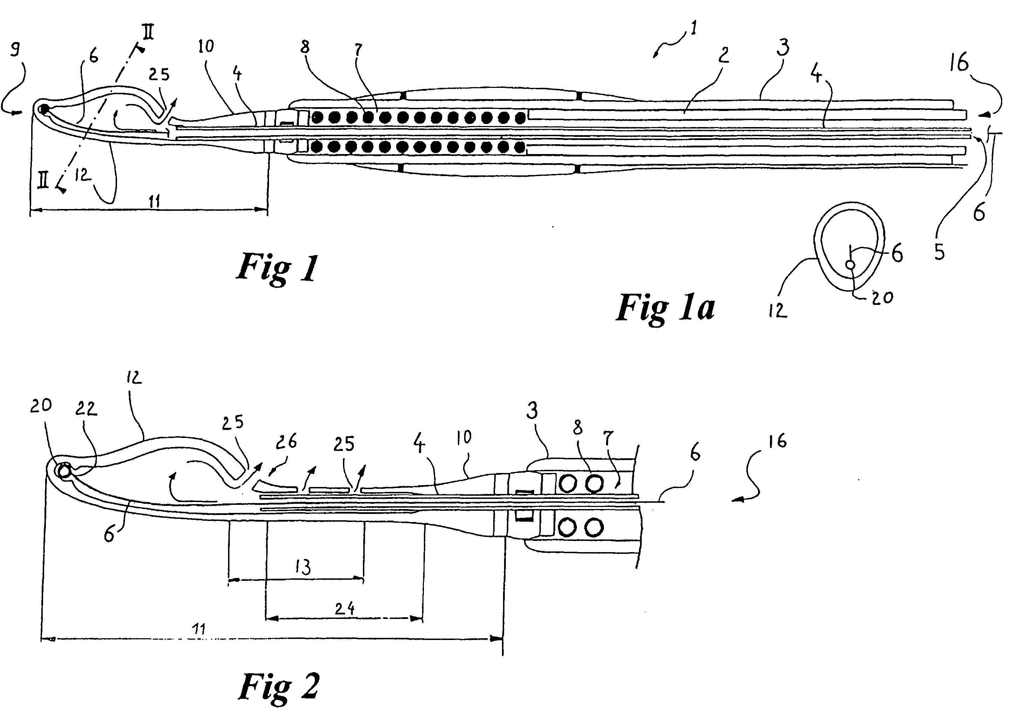

As can be seen from Fig. 1, the device 1 according to the invention includes a central stent pusher part 2 surrounded by a stent releasing part 3.

The stent pusher part 2 comprises a microcatheter 4 provided with an axial lumen 5. A microguide wire 6 extends along the axial lumen 5. A stent loading cavity 7 able to contain a stent 8 - in the present case, a self-expanding stent - is provided near the distal end 9 of the stent pusher part 2.

The distal end 9 of the device 1 bears an atraumatic tip 10 which is prolonged by a tip balloon part 11 comprising an inflatable occlusive balloon 12 and a fluid releasing section 13. The tip balloon part 11 is in operation connected via the axial lumen 5 and a Y-adapter (not shown) to injection means (not shown) placed towards the proximal end 16 of the device 1.

This injection means are able to provide a continuous flow of a physiologically acceptable fluid at a predetermined rate. A stop-lock part serves to lock the stent pusher part 2 in a fixed relative position with respect to the stent releasing part 3.

When the device 1 is inserted in a body, the tip balloon part 11 leads the device 1 through the vascular system and through possible stenoses. Indeed, it is possible to change the shape of the occlusive balloon 12 when it is in the deflated state by advancing the microguide wire 6 more or less into the tip balloon part 11.

To this end, the guide wire 6 is anchored into a tip pouch 20 of the balloon 12 by a small spherical ball 22. Relative advancement of the microguide wire 6 will therefore induce bending of the wire tip.

A marker (for instance a colour marker) at the proximal side of the guide wire 6 permits a control of the maximal allowed advancement in relation to the position of the Y-adapter on the stent pusher part 2.

The tip balloon part 11 of the device 1 serves as a barrier which prevents plaque debris from embolizing the cerebral arterial circulation, by temporary occluding the main arterial axis (i.e. the carotid artery or the vertebral artery) upstream with respect to the lesion to be cured.

The occlusive balloon 12 can be inflated to a diameter of about 5-6 mm and at a length of about 10-12 mm, thus hermetically closing the artery while avoiding over distension thereof.

The presence of a fluid-releasing section 13 on the proximal face of the balloon 12 - or just there behind - provides for an efficient flushing action represented by tiny arrows on the figures), far from the limited possibilities of classical methods.

The inflation of the occlusive balloon 12 occurs independent of the flushing function at a pressure range of approximately 300-800 mm Hg. Flushing of the section of artery upstream from the occlusive balloon 12 occurs at increased pressures, allowing overflow fluid to escape through the fluid-releasing section 13 in the balloon neck area at a flow rate sufficient to force back the blood, possibly laden with particles, up to an upstream vascular bifurcation. The flow rate can be about 1-2 cm³/sec.

This flush action causes a continuous cleaning of the vascular volume in the artery upstream from the occlusive balloon 12 whenever the balloon pressure reaches a given value, thus activating a controlled leak via the fluid-releasing section 13.

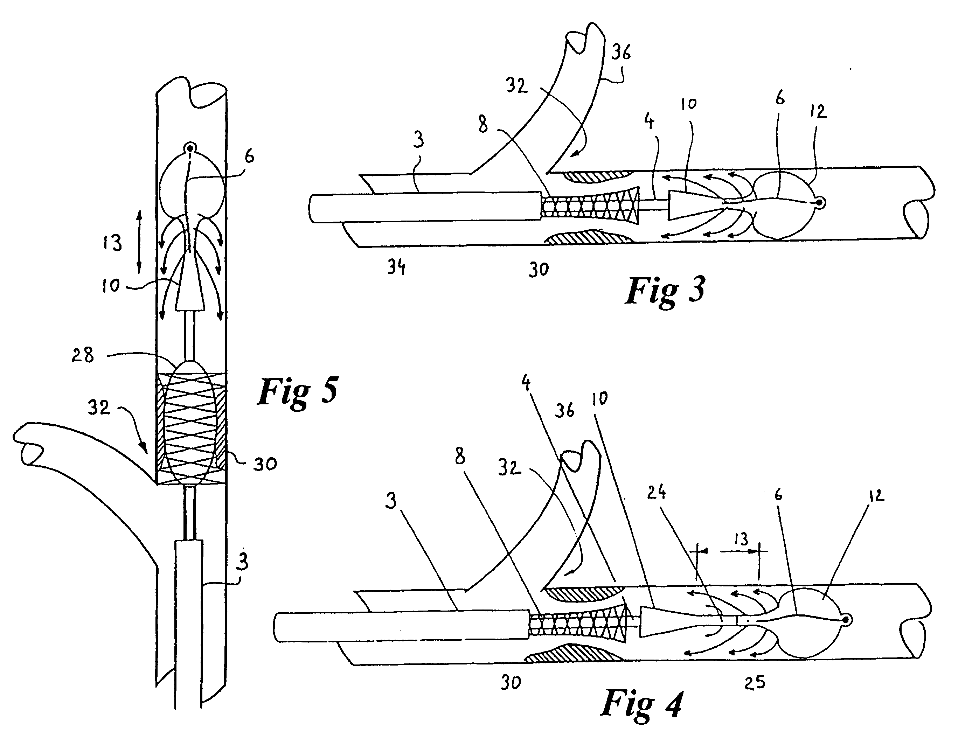

The tip balloon part 11 can be adjacent the atraumatic tip 10, as shown on Fig. 1, or placed at the tip of a cane 24 protruding from this atraumatic tip 10, as represented on Figs. 2 and 4.

Figs. 3 and 4 display various embodiments of the fluid-releasing section 13, the occlusion balloon 12 having been inflated by an increase of the pressure released by injection means. The fluid begins to escape from the balloon 12 at a predetermined rate through calibrated pin-holes 25 provided at the proximal side of the occlusive balloon 12 and/or of the neck 26 thereof.

The holes are preferably designed so that the fluid cannot escape unless the pressure reaches a trigger value.

The fluid-releasing section 13 can also extend on the cane 24 as shown on Fig. 4.

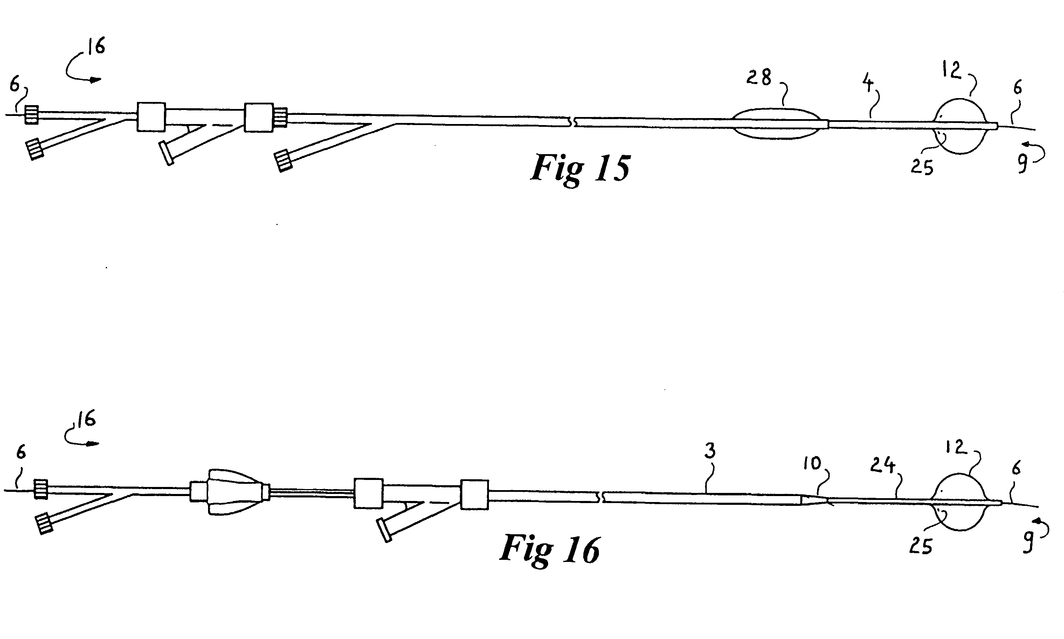

Of course, the device can also be guided in a conventional manner along the microguide wire 5 as can be seen on Fig. 15.

Fig. 15 further displays the proximal end of the device and various Y-adapters for connecting i.a. the axial lumens 5 to the injection means. In this case, the axial lumen 5 extends through the tip pouch 20 and comprises a separated channel to feed the tip balloon 12.

The stent releasing part 3 can be designed so as to accommodate a self-expanding stent as shown for instance on Figs. 2, 3 or a balloon-expandable stent, as shown in Fig. 5; in this case, the stent-releasing part includes a dilatation balloon 28.

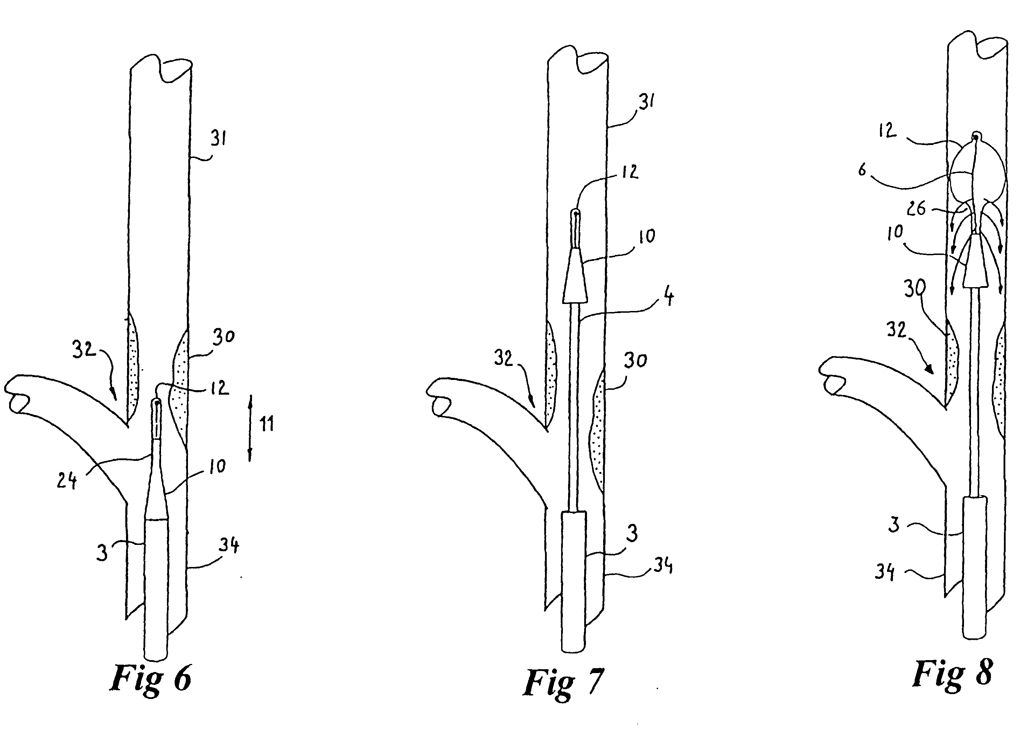

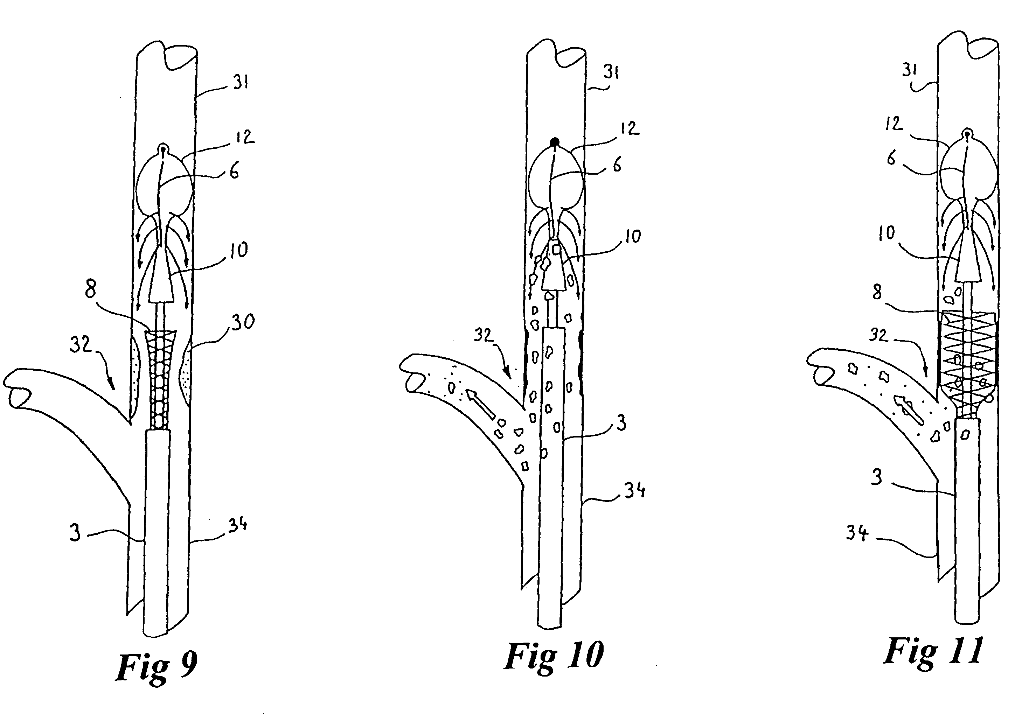

Figs. 6 to 12 illustrate diagrammatically the various steps of the safe method according to the invention which may now be applied by using the above-described device 1 according to the invention.

The distal end 9 of the device 1 having been inserted in the vascular system according to a known method (generally from the femoral artery), it is driven easily up to the site to be cured, in the present case a stenosis 30 in the inner carotid 31, downstream with respect to the bifurcation 32 of the common carotid 34.

The microcatheter 4 is then extended up to a segment of the inner carotid 31 beyond the stenosis 30. The injection means are then activated, at a predetermined rate, so that the pressure of the fluid increases in the axial lumen 5, thereby causing the balloon 12 to expand.

When the balloon 12 reaches a diameter substantially equal to that of the inner carotid 31, its internal pressure begins to rise, causing the pin-holes 25 of the fluid-releasing section 13 to open and, flushing backwards the fluid and the blood volume trapped behind the balloon 12. The brain being in such a way protected from particles liable to escape from the stenosis 30, the physician can immediately begin to proceed with placement of a stent 8, which can be either a self-expanding stent 8 constricted in the stent loading area 7, or a balloon-expandable stent. In the latter case, the placement implies activating a dilatation balloon 28 as can be seen on Fig. 5.

During this operation, the tip balloon part 11 goes on flushing backwards the blood laden with particles up to the carotid bifurcation 32, this laden blood being diverted through the outer carotid to other organs where it can cause no harm.

It should be stressed that debris and particles are completely flushed away, since there remains no place behind the balloon 12 where they could stay.

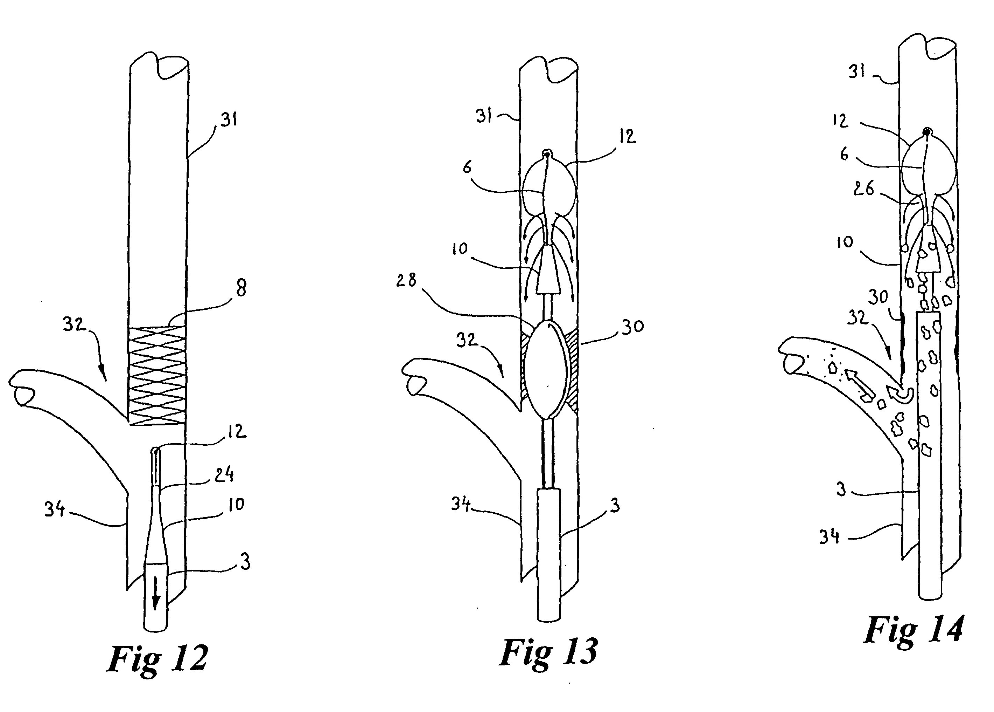

If necessary, depending on the state of the stenosis 30, it is possible to proceed within the allowed time, preliminary to the insertion of the stent 8, as shown in Fig. 13, to a widening of the section to be cured, plaque debris being still carried away by the constant flushing of the artery.

Another feature of the present device becomes apparent when comparing Fig. 4 and Fig. 13: the dilatation balloon 28 shown in the latter is able to slide along the microcatheter 4 and can accordingly reach at will any part of the artery wall to be cured upstream from the occlusive balloon 12. To allow such an axial displacement, the balloon 28 is connected to a balloon pusher placed between the microcatheter 4 and the stent releasing part 3.

During the whole operation, the duly monitored injection means go on feeding the occlusive balloon 12 and the fluid-releasing section 13 at a rate sufficient to keep the balloon 12 safely inflated and to provide a sufficient flow rate to drive the blood to the bifurcation 32.

The operation being carried through, the balloon 12 can be instantaneously deflated and withdrawn through the implanted stent 8.

The stent-releasing part 3 may comprise an outer tubing or, to reduce the diameter, a single outer tubing making it possible to directly release the stent 8 at its predetermined place.

Advantageously, the outer tubing can comprise radio-visible markers allowing the stent 8 to be placed accurately at its required place.

An advantage of the device of the invention is that, once the occlusive balloon 12 has been placed, it is possible, according on the circumstances, to carry out without delay a wide variety of operations on the site to be cured. If necessary, it is even possible, in a matter of seconds, to replace a part of the device without disturbing the blocking function of the tip part. The time so spared can make the difference for the patient.

The device can be also provided as a kit of parts to be assembled, which allows the operator to select and assemble at the very moment of the operation e.g. the kind of stent he feels to be the more adapted to the circumstances and the corresponding stent releasing part.

Fig. 16 displays another embodiment of the device, which in the present case includes a tip balloon part 11 and a dilatation balloon 28 axially displaceable relative to same, but devoid of stent releasing part 3.

The embodiment of the device shown in Fig. 16 can either be used as such in particular cases wherein it is not compulsory to place as stent after having cured the stenasis or as part of a kit to perform the preliminary operations of an intervention, bearing in mind that an adequate stent releasing part can be fitted thereon within a few seconds without interrupting the blocking action of the occlusive balloon 12.

In short, the invention can be described as follows :

The present invention relates to a device for protected angioplasty, intended for the implantation of luminal endoprosthesis (or stent) in critical areas such as carotid or vertebral arteries, where protection of downstream-situated organs is highly desirable. The device comprises a central stent pusher part comprising a microcatheter bearing at its distal end an atraumatic tip, said atraumatic tip being prolonged by a tip balloon part comprising an inflatable occlusive balloon which may be inflated with a physiologically acceptable fluid at predetermined rates, a fluid-releasing section extending at the proximal side of the occlusive balloon, said releasing section being able to release the fluid from the balloon into an upstream section of the vessel when the pressure of said fluid reaches a predetermined level.

## Claims

1. A device (1) for the implantation of expandable stents (8) in a vascular vessel, allowing for temporary protection of downstream organs, comprising
- a central part (2) provided with an axial lumen (5), for a microguide wire (6), **and comprising a microcatheter (4)** bearing at its distal end (9) an atraumatic tip (10),
- a tip balloon part (11) comprising an inflatable occlusive balloon (12) hermetically connectable via the axial lumen (5) to injection means able to feed said balloon (12) with a physiologically acceptable fluid,
**characterized in that**
- the central part comprises a stent pusher part (2) surrounded by a stent-releasing part (3),
- a stent loading cavity (7), containing a stent (8) in radially contracted state, extends near the distal end of the stent pusher part (2),
- a fluid-releasing section (13) extends at the proximal side of the occlusive balloon (12), said releasing section (13) releasing in operation **the same** physiologically acceptable fluid **fed at predetermined rates** from the inflated occlusive balloon (12) into an upstream section of the vessel **when the pressure of said fluid reaches a predetermined level.**

2. A device (1) according to claim 1 **characterized in that** it comprises a dilatation balloon (28) axially displaceable relative to the tip balloon (12).

3. A device according to claim 1, **characterized in that** the stent (8) is a self-expanding stent, which is maintained in place in the stent loading cavity (7) by a surrounding shell.

4. A device according to claim 2 **characterized in that** the stent (8) is a balloon-dilatable stent, the stent-releasing part (3) comprising a dilatation balloon (28).

5. A device according to any one of the preceding claims, **characterized in that** the fluid-releasing section (13) extends at the proximal end of the occlusive balloon (12).

6. A device according to any one of the preceding claims, **characterized in that** the fluid-releasing section (13) extends on the neck of the balloon (12).

7. A device according to any one of the preceding claims, **characterized in that** the fluid-releasing section (13) extends on a cane (24) supporting the occlusive balloon (12).

8. A device according to any one of the preceding claims, **characterized in that** the microguide wire (6) is anchored at the distal side of the occlusive balloon (12).

9. A device according to claim 8, **characterized in that** the wire (6) is terminated by a ball (22) inserted in a pouch (20) provided at said distal side.

10. A device according to any one of the preceding claims, **characterized in that** the fluid-releasing section is designed so that the fluid cannot escape unless its pressure reaches a trigger value.

## Patentansprüche

1. Vorrichtung (1) zur Implantation von expandierbaren Stents (8) in einem Gefäß zum vorübergehenden Schutz von dahinter liegenden Organen, umfassend einen mittleren Teil (2) mit einem axialen Lumen (5) für einen Mikroführungsdraht (6) und einen Mikrokatheter (4) mit einer atraumatischen Spitze (10) an seinem distalen Ende (9), ein Spitzenballonteil (11) mit einem aufblasbaren Okklusionsballon (12), der über das axiale Lumen (5) hermetisch dichtend mit einem Injektionsmittel verbunden werden kann, das dem Ballon (12) eine physiologisch annehmbare Flüssigkeit zuführen kann, **dadurch gekennzeichnet, dass** der mittlere Teil einen Stentschubteil (2) umfasst, der von einem Stentfreigabeteil (3) umgeben ist, sich eine Stentladehöhle (7), die einen Stent (8) im radial zusammengezogenen Zustand enthält, in der Nähe des distalen Endes des Stentschubteils (2) erstreckt, sich ein Flüssigkeitsfreisetzungsabschnitt (13) auf der proximalen Seite des Okklusionsballons (12) erstreckt, wobei dieser Freisetzungsabschnitt (13) im Betrieb die gleiche physiologisch annehmbare Flüssigkeit freisetzt, die mit vorbestimmten Geschwindigkeiten vom aufgeblasenen Okklusionsballon (12) in einen stromaufwärts gelegenen Abschnitt des Gefäßes zugeführt wird, wenn der Druck der Flüssigkeit eine vorbestimmte Höhe erreicht.

2. Vorrichtung (1) nach Anspruch 1, **dadurch gekennzeichnet, dass** sie einen Dilatationsballon (28) umfasst, der relativ zum Spitzenballon (12) axial verschiebbar ist.

3. Vorrichtung nach Anspruch 1, **dadurch gekennzeichnet, dass** der Stent (8) ein selbstexpandierbarer Stent ist, der von einer umgebenden Schale in der Stentladehöhle (7) gehalten wird.

4. Vorrichtung nach Anspruch 2, **dadurch gekennzeichnet, dass** der Stent (8) ein ballondilatierbarer Stent ist, wobei der Stentfreigabeteil (3) einen Dilatationsballon (28) umfasst.

5. Vorrichtung nach einem der vorhergehenden Ansprüche, **dadurch gekennzeichnet, dass** sich der Flüssigkeitsfreisetzungsabschnitt (13) am proximalen Ende des Okklusionsballons (12) erstreckt.

6. Vorrichtung nach einem der vorhergehenden Ansprüche, **dadurch gekennzeichnet, dass** sich der Flüssigkeitsfreisetzungsabschnitt (13) am Hals des Ballons (12) erstreckt.

7. Vorrichtung nach einem der vorhergehenden Ansprüche, **dadurch gekennzeichnet, dass** sich der Flüssigkeitsfreisetzungsabschnitt (13) auf einem den Okklusionsballon (12) unterstützenden Stock (24) erstreckt.

8. Vorrichtung nach einem der vorhergehenden Ansprüche, **dadurch gekennzeichnet, dass** der Mikroführungsdraht (6) auf der distalen Seite des Okklusionsballons (12) verankert ist.

9. Vorrichtung nach Anspruch 8, **dadurch gekennzeichnet, dass** der Draht (6) von einer Kugel (22) abgeschlossen wird, die in einen Beutel (20) auf der distalen Seite eingesetzt ist.

10. Vorrichtung nach einem der vorhergehenden Ansprüche, **dadurch gekennzeichnet, dass** der Flüssigkeitsfreisetzungsabschnitt so konstruiert ist, dass die Flüssigkeit nur austreten kann, wenn der Druck einen bestimmten Wert erreicht.

## Revendications

1. Dispositif (1) pour l'implantation de stents expansibles (8) dans un vaisseau vasculaire, permettant la protection temporaire d'organes situés en aval, comprenant :
- une partie centrale (2) pourvue d'un lumen axial (5) pour un fil métallique de microguidage (6), **et comprenant un microcatéther (4)** portant, à son extrémité distale (9), une pointe atraumatique (10),
- une partie de pointe à ballonnet (11) comprenant un ballonnet gonflable occlusif (12) qui peut être raccordé hermétiquement, via le lumen axial (5), à des moyens d'injection capables d'alimenter ledit ballonnet (12) avec un fluide physiologiquement acceptable,
**caractérisé en ce que**
- la partie centrale comprend une partie pousse-stent (2) entourée d'une partie de libération de stent (3),
- une cavité de chargement de stent (7), contenant un stent (8) à l'état radialement contracté, s'étend près de l'extrémité distale de la partie pousse-stent (2),
- une section de libération de fluide (13) s'étend sur le côté proximal du ballon occlusif (12), ladite section de libération (13) libérant en service **le même** fluide physiologiquement acceptable **acheminé à des débits prédéterminés** depuis le ballonnet occlusif gonflé (12) dans une section amont du vaisseau **lorsque la pression dudit fluide atteint un niveau prédéterminé.**

2. Dispositif (1) selon la revendication 1, **caractérisé en ce qu'**il comprend un ballonnet de dilatation (28) axialement déplaçable par rapport au ballonnet de pointe (12).

3. Dispositif (1) selon la revendication 1, **caractérisé en ce que** le stent (8) est un stent autodilatant qui est maintenu en place dans la cavité de chargement de stent (7) par une coque environnante.

4. Dispositif selon la revendication 2, **caractérisé en ce que** le stent (8) est un stent dilatable par un ballonnet, la partie de libération de stent (3) comprenant un ballonnet de dilatation (28).

5. Dispositif selon l'une quelconque des revendications précédentes, **caractérisé en ce que** la section de libération de fluide (13) s'étend à l'extrémité proximale du ballon occlusif (12).

6. Dispositif selon l'une quelconque des revendications précédentes, **caractérisé en ce que** la section de libération de fluide (13) s'étend sur le col du ballonnet (12).

7. Dispositif selon l'une quelconque des revendications précédentes, **caractérisé en ce** la section de libération de fluide (13) s'étend sur une tige (24) supportant le ballonnet occlusif (12).

8. Dispositif selon l'une quelconque des revendications précédentes, **caractérisé en ce** le fil métallique de microguidage (6) est ancré sur le côté distal du ballonnet occlusif (12).

9. Dispositif selon la revendication 8, **caractérisé en ce que** le fil métallique (6) se termine par une bille (22) insérée dans un sac (20) ménagé sur ledit côté distal.

10. Dispositif selon l'une quelconque des revendications précédentes, **caractérisé en ce** la section de libération de fluide est conçue de sorte que le fluide ne puisse s'échapper à moins que sa pression atteigne une valeur de déclenchement.
